# EUROPEAN PATENT SPECIFICATION

(11) **EP 3 065 801 B1**
(45) Date of publication and mention of the grant of the patent: **14.08.2019**
(21) Application number: 14815405.7
(22) Date of filing: 05.11.2014
(51) Int. Cl.: A61M 5/24, A61M 5/28

(54) **A PHARMACEUTICAL COMPONENT-MIXING DELIVERY ASSEMBLY**
AUSGABEVORRICHTUNG MIT MISCHUNG VON PHARMAZEUTISCHEN BESTANDTEILEN
ENSEMBLE D'ADMINISTRATION MÉLANGEANT LES ÉLÉMENTS PHARMACEUTIQUES

(30) Priority: 05.11.2013 GB 201319467; 25.07.2014 GB 201413208; 01.08.2014 GB 201413715
(43) Date of publication of application: 14.09.2016
(73) Proprietor: 3P Innovation Limited, Warwick Warwickshire CV34 6UQ (GB)
(72) Inventor: BAILEY, Thomas, Warwick Warwickshire CV34 5PZ (GB); SEAWARD, David, Warwick Warwickshire CV34 5PZ (GB)
(74) Representative: Gilholm, Stephen Philip
(86) International application number: PCT/GB2014/053286
(87) International publication number: WO 2015/067935

(56) References cited:
- WO-A2-96/29106
- DE-A1- 1 909 794
- FR-A1- 2 799 654
- US-A- 3 326 215
- US-A- 3 685 514
- US-A- 3 785 379
- US-A- 4 233 975
- US-A- 5 281 198

## Description

### Field of the Invention

The present invention relates to a novel syringe suitable for mixing a pair of components.

More particularly, the invention relates to a novel syringe suitable for mixing a two component system, such as a solid medicament and a liquid, liquid/ liquid components, viscous/non-viscous components, etc. The present invention also relates to methods related thereto.

### Background to the Invention

Many medicaments are unsuitable for the long term storage as a liquid solution because they may be unstable when mixed into a solution and thus have a shorter shelf-life than other forms. Such medicaments may be reconstituted with a diluent or carrier, usually a liquid, prior to administration. Thus, liquid or solid medicaments may be stored in a sealed vial prior to reconstitution. Increasingly, prefilled syringes capable of separate storage of at least two different components are being used. Such syringes usually comprise a medicinal component stored in a first chamber and a dissolving or dispersing agent in a second chamber. The components are kept apart until use.

Dual chamber syringes have been developed which can suitably be used with powdered medicaments and a suitable liquid. Such dual chambers may be used for the administration of "biopharmaceuticals", such as recombinant proteins; and the like.

One example of a commercially successful dual chamber syringe is the Vetter Lyo-Ject® dual chamber syringe, which is known to be used with recombinant anti-haemophilic factor. The recombinant anti-haemophilic factor is lyophilised *in situ* in the syringe. The Vetter syringe is described in US Patent No. 6,419,656 and comprises a tubular body extending along an axis; a plunger axially slidable in the body and a free piston slidable in the body forward of the plunger. The plunger subdivides the body into a front compartment, forward of the plunger, and a rear compartment between the plunger and the piston. The body is also provided with a bypass passage forward of the piston. In use, a lyophilised medicament is located in the front compartment and a liquid is located in the rear compartment. When the plunger is depressed and the second compartment is drawn alongside the bypass chamber, the liquid is able to flow into the first compartment and solubilise the medicament or form a suspension of the medicament.

However, one disadvantage of dual chamber syringes, such as the Vetter Lyo-Ject® syringe, is that it is expensive to manufacture. Also producing a lyophilised powdered medicament in situ in the syringe is difficult and does not lend itself to rapid and large scale manufacturing techniques.

Other dual chamber syringes are known. For example, US Patent No. 5,281,198 describes a syringe assembly suitable for multiple pharmaceutical components, such as a lyophilized medicament component and a diluent component contained within first and second cartridges fitted with a movable piston. The second cartridge is forced into the interior of the first cartridge causing a spike or needle assembly to couple the two cartridges and causing the contents of the first cartridge to be driven into the second cartridge, thereby mixing them. A ratchet plunger is used to drive a second piston, the distance that the ratchet plunger moves determining the dose administered.

French Patent application No. 2799654 describes a syringe arrangement wherein a non-liquid part of a formulation to be injected is packed in the body of the syringe and a liquid reconstitution part is packed separately in a reservoir which can form the injection rod of the piston of the syringe. The transfer system between the two parts (the body of the syringe and the reservoir) is provided by a needle, a tube or a rod which has the characteristic of entering the non-liquid reservoir through the piston which serves for the injection of the reconstituted form. An assembly with the features of the preamble of claim 1 is known from WO96/29106.

However, one disadvantage with such devices is that small particles may be generated when the spike or needle assembly penetrates or pierces a septum dividing a first and second cartridge. Furthermore, the user may experience considerable resistance or friction in pushing the plunger to pierce the septum. It is also important that the sterility of the syringe is maintained until the device is ready to be used.

### Summary of the Invention

We have now found a device that overcomes or mitigates the problems associated with the prior art devices. More particularly, we have found a novel dual syringe assembly which avoids the need to pierce a septum. Furthermore, the novel device of the present invention can suitably utilise conventional standard syringe components, avoiding the need for a bypass chamber to be built into the body of a syringe.

Thus, according to a first aspect of the present invention there is provided a pharmaceutical component-mixing delivery assembly (1) with the features of claim 1.

The inclusion of a terminal rib is advantageous in that, *inter alia,* it allows the sterility of the delivery assembly to be maintained when the device is primed before injection.

Furthermore, the non-invasive valve component, e.g. a piston, is generally elongated. The region of the second cartridge that contains the second pharmaceutical component will generally comprise an aseptic mixing chamber, whilst the inner wall of the second cartridge outside of the mixing chamber and beyond the terminal rib of the piston, will generally be non-aseptic. In use, as the plunger of the delivery assembly is depressed and the piston moves towards the inlet end of the second cartridge, the mixing chamber essentially expands longitudinally. Thus, the length of the piston is desirably chosen so that as the piston moves, the mixing chamber never comes into contact with the non-aseptic walls of the second cartridge and the sterility of the mixing chamber is maintained at all times. Therefore, the length of the piston will be dictated by how far back along the second cartridge it has to move, which will in turn be dictated by the volume required for the mixing chamber.

In a particular aspect of the invention the non-invasive valve component may comprise a ball valve, a flow through stopper component or a T-shaped stopper component.

The pharmaceutical component-mixing delivery assembly will generally comprise a syringe assembly. Thus, the pharmaceutical component-mixing delivery assembly hereinbefore described may include a needle, e.g. a hypodermic needle, attached to the outlet end of the second cartridge.

One of the first and second pharmaceutical components may comprise a solid therapeutically active agent and the other is a liquid capable of solubilising or forming a suspension with the solid therapeutically active agent. In a particular aspect of the invention the first pharmaceutical component comprises a liquid and the second pharmaceutical component comprises a solid therapeutically active agent. However, it will be understood by the person skilled in the art that the first and second pharmaceutical components may comprise liquid/ liquid components, viscous/non-viscous components, etc.

The use of a non-invasive valve as hereinbefore described is advantageous in that, *inter alia,* conventional syringe bodies and/or plungers may be utilised, significantly reducing the expense of the syringe assembly as a whole. It also avoids, for example, the need for an elastomeric septum.

In one aspect of the invention the non-invasive valve component may comprise a ball valve. Such a ball valve component may comprise one or more balls located within the outlet of the first cartridge, such that the ball(s) acts to seal the first cartridge. The ball valve component includes a substantially hollow apertured piston suitable for receiving the ball from the first cartridge.

More specifically, the ball valve component may comprise a substantially hollow apertured piston with a first end adjacent the outlet end of the first cartridge and a second end adjacent the inlet end of the second cartridge. The hollow apertured piston will comprise side walls which generally form a seal against the inner walls of the second cartridge.

The first end of the piston may comprise means for locating the outlet end of the first cartridge. For example, such means may comprise a substantially circumferential ridge. The second end of the piston may comprise one or more apertures which, when in use, are dimensioned to allow fluid to flow.

A single ball or a plurality, e.g. two or three balls, may be located within the outlet of the first cartridge, such that the ball(s) acts to seal the first cartridge.

In use, when the plunger is depressed within the first cartridge the fluid pressure will force the ball valve to open allowing fluid to flow from the first cartridge to the second cartridge enabling mixing to occur.

In another aspect of the invention the non-invasive valve component may comprise a ball valve as hereinbefore described. However, the second end of the piston may comprise a separate plug component which is provided with one or more apertures which, when in use, are dimensioned to allow fluid to flow. In addition, the plug component may be provided with means for displacing the ball from the outlet of the first cartridge allowing fluid to flow from the first cartridge to the second cartridge enabling mixing to occur. Thus, in accordance with this aspect of the invention there is provided a pharmaceutical component-mixing delivery assembly as hereinbefore described wherein the ball valve component includes a plug component which is provided with one or more apertures and which is provided with means for displacing the ball from the outlet of the first cartridge. One example of such displacement means is a plug provided with a protruding spigot.

In another aspect of the invention the non-invasive valve component may comprise a flow through stopper component. The flow through stopper component may comprise a stopper which is provided with conduit means. Said conduit means may comprise an internal conduit within the stopper or alternatively, may comprise an external conduit, that is, a conduit between the walls of the piston and the stopper.

One example of a flow through stopper component with an internal conduit is a T-valve. Such a T-valve component may comprise a substantially hollow apertured piston with a first end adjacent the outlet end of the first cartridge and a second end adjacent the inlet end of the second cartridge. The hollow apertured piston will comprise side walls which generally form a seal against the inner walls of the second cartridge as hereinbefore described.

The first end of the piston may comprise means for locating the outlet end of the first cartridge. For example, such means may comprise a substantially circumferential ridge. The piston also houses a stopper which has a flow through capability and which seals the outlet end of the first cartridge. Said stopper may comprise an internal conduit which is substantially coaxial with the longitudinal axis of the first cartridge, but which is provided with one or more branches which seal against the side walls of the piston.

In use, when the plunger is depressed within the first cartridge the fluid pressure will displace the flow through stopper such that the one or more branches of the conduit are no longer sealed against the side walls of the piston allowing fluid to flow from the first cartridge to the second cartridge enabling mixing to occur. Optionally, a second stopper may be provided adjacent the inlet of the second cartridge. The second stopper may also be provided with one or more branched conduits, which act in a substantially similar manner to the first.

In any of the embodiments described herein the side walls of the non-invasive valve component may be provided with one or more external ribs to improve the seal formed between the non-invasive valve component and the second cartridge. Thus, for example, when the non-invasive valve component comprises a piston, the piston may be ribbed, i.e. is provided with one or more external ribs, to improve the seal formed between the piston and the inner walls of the second cartridge. Preferably the piston is provided with one or more circumferential ribs. It is especially preferred for the piston to be provided with a plurality of circumferential ribs, e.g. 2, 3 or 4 ribs.

An alternative example of a flow through stopper component with an external conduit is an "annular valve". Although an "annular valve" possesses a similar functionality to the aforementioned T-valve, the "annular valve" stopper itself does not comprise an internal conduit.

Adjacent the first cartridge the internal walls of the piston are configured to form a snug fit with the stopper. In this first position, the stopper seals the first cartridge. The piston also provides a second position wherein the stopper forms a loose fit, such a conduit external to the stopper is formed between the stopper and the piston, such that fluid may pass through the conduit, between the walls of the piston and the stopper.

Thus, according to this aspect of the invention there is provided a pharmaceutical component-mixing delivery assembly as hereinbefore described wherein the "annular valve" comprises a piston configured to form a snug fit with a stopper which seals the first cartridge; and
wherein the piston provides a second position wherein the stopper forms a loose fit, such that fluid may pass between the walls of the piston and the stopper.

The use of a T-valve is advantageous, *inter alia,* in that:
- It provides guidance for the piston in the hollow stopper (the plug cannot tilt)
- Aids assembly - physical stop
- Prevents the piston being accidently drawn back into the syringe
- Ribs provide enhanced sealing in the neck of the of the cartridge
- The larger diameter part is held by the hollow stopper which provides additional retention of the piston

According to a further aspect of the present invention there is provided a method of operating a pharmaceutical component-mixing delivery assembly (1) according to claim 10.

According to a particular aspect of the method of the invention the non-invasive valve component is provided with a terminal rib to improve the seal formed between the non-invasive valve component and the second cartridge.

The pharmaceutical component-mixing delivery assembly will generally comprise a syringe assembly. Thus, the method of administering a therapeutically active agent to a patient will generally comprises an additional step of attaching a needle, e.g. a hypodermic needle, to the outlet end of the second cartridge, followed by administering the mixed pharmaceutical components to a patient.

In the aforementioned methods preferably one of the first and second pharmaceutical components comprises a solid therapeutically active agent and the other is a liquid capable of solubilising or forming a suspension with the solid therapeutically active agent. In a particular aspect of the invention the first pharmaceutical component comprises a liquid and the second pharmaceutical component comprises a solid therapeutically active agent. Alternatively, the first and second pharmaceutical components may comprise liquid/ liquid components, viscous/non-viscous components, etc.

In another embodiment of the present invention resistance or friction may be minimised for the user by providing a plunger with an external screw thread. In this particular embodiment the plunger is desirably threaded along a predetermined portion of its length. It is within the scope of the present invention for the plunger to be threaded along the whole of its length, however, it is preferred that the plunger is only partially threaded, i.e. threaded along only a portion of its length. Thus, the user may screw the plunger to bring the contents of the first and second cartridges into contact, since this is generally where the user experiences most resistance. However, the remainder of the shaft of the plunger may desirably be unthreaded so that a conventional push action may be used for administration of the medicament solution to a patient.

The use of a threaded, or partially threaded, plunger as hereinbefore described may also be advantageous in that it may be used to provide a sealed system, improving sterility of the delivery device. Thus, a frangible seal may be provided around the shaft of the plunger, for example, within the body of the first cartridge, such that twisting of the plunger by the user can cause the frangible seal to break.

The use of a threaded plunger may suitably be used with any of the aforementioned syringe embodiments hereinbefore described. However, it will be understood by the person skilled in the art that such a threaded plunger arrangement is novel *per se* and may therefore be suitably applied to a conventionally known invasive valve component, such as a needle piercing an elastomeric septum. Desirably, the pharmaceutical component-mixing delivery assembly according to this aspect of the invention will generally comprise a sealed unit, e.g. an outer casing which house the first and second cartridges.

A particular advantage of the use of a threaded plunger is that a seal may be placed around the end of the first cartridge by, i.e. between the first cartridge and the outer casing.

Thus, according to this further aspect of the present invention there is provided a pharmaceutical component-mixing delivery assembly comprising:
an outer casing is provided which houses a first and a second cartridge;
the first and second cartridges being dimensioned so that they are movable relative to one another such that the first cartridge is slidable within the second cartridge from a pre-mixed position to a post-mixed position;
the first cartridge comprising a body provided with an outlet end, the first cartridge being fitted with an at least partially threaded plunger the end of which protrudes from the outer casing, said first cartridge containing a first pharmaceutical component;
the second cartridge comprising a body provided with an inlet end and an outlet end, the second cartridge containing a second pharmaceutical component;
wherein the first and second cartridges are separated by a valve component; and
a frangible seal is provided between the outer casing and the first cartridge.

In use, when the at least partially threaded plunger is twisted the frangible seal is broken; then as the at least partially threaded plunger and the cartridge are pushed towards the second cartridge the first cartridge disengages from the seal. The use of a frangible seal enables the device to remain sterile during storage, whilst the disengagement of the frangible seal from the first cartridge reduces friction and lowers force required to complete the administration of the therapeutically active agent.

According to this aspect of the invention the valve component may comprise a non-invasive valve as hereinbefore described or a conventionally known valve, such as a needle valve or a bypass valve.

In a further aspect of the invention there is provided a method of administering a therapeutically active agent to a patient which comprises operating a pharmaceutical component-mixing delivery assembly holding first and second pharmaceutical components; said delivery assembly comprising:
an outer casing is provided which houses a first and a second cartridge;
the first and second cartridges being dimensioned so that they are movable relative to one another such that the first cartridge is slidable within the second cartridge from a pre-mixed position to a post-mixed position;
the first cartridge comprising a body provided with an outlet end, the first cartridge being fitted with an at least partially threaded plunger the end of which protrudes from the outer casing, said first cartridge containing a first pharmaceutical component;
the second cartridge comprising a body provided with an inlet end and an outlet end, the second cartridge containing a second pharmaceutical component;
wherein the first and second cartridges are separated by a valve component; and
a frangible seal is provided between the outer casing and the first cartridge.

The method of operation according to this aspect of the invention will generally comprise first turning the at least partially threaded plunger causing the frangible seal to break and disengage from the first cartridge and subsequently pressing the plunger to administer the mixed pharmaceutical components to a patient.

The term "non-invasive valve" will be understood by the person skilled in the art. However, for the avoidance of doubt, the term should be construed so as not to include a component wherein, for example, a needle is utilised to pierce a septum and the like. Furthermore, the term "non-invasive valve component" should be construed as meaning that the valve is made up entirely of the component, i.e. it does not utilise the wall of the syringe or cartridge (which is distinct from, for example, the Vetter syringe described in US Patent No. 6,419,656).

According to a yet further aspect of the invention there is provided a kit suitable for mixing and delivery of pharmaceutical components, said kit comprising:
a first cartridge and a second cartridge;
the first and second cartridges being dimensioned so that when placed together they are movable relative to one another such that the first cartridge is slidable within the second cartridge from a pre-mixed position to a post-mixed position;
the first cartridge comprising a body provided with an outlet end, the first cartridge being fitted with plunger and containing a first pharmaceutical component;
the second cartridge comprising a body provided with an inlet end and an outlet end, the second cartridge containing a second pharmaceutical component; and
wherein when the first and second cartridges are placed together they are separated by a non-invasive valve component.

There is also provided a kit suitable for mixing and delivery of pharmaceutical components, said kit comprising:
an outer casing which houses a first and a second cartridge;
the first and second cartridges being dimensioned so that they are movable relative to one another such that the first cartridge is slidable within the second cartridge from a pre-mixed position to a post-mixed position;
the first cartridge comprising a body provided with an outlet end, the first cartridge being fitted with an at least partially threaded plunger the end of which protrudes from the outer casing, said first cartridge containing a first pharmaceutical component;
the second cartridge comprising a body provided with an inlet end and an outlet end, the second cartridge containing a second pharmaceutical component;
wherein the first and second cartridges are separated by a valve component; and
a frangible seal is provided between the outer casing and the first cartridge.

The invention further provides the use of a pharmaceutical component-mixing delivery assembly comprising:
a first cartridge and a second cartridge;
the first and second cartridges being dimensioned so that they are movable relative to one another such that the first cartridge is slidable within the second cartridge from a pre-mixed position to a post-mixed position;
the first cartridge comprising a body provided with an outlet end, the first cartridge being fitted with plunger and containing a first pharmaceutical component;
the second cartridge comprising a body provided with an inlet end and an outlet end, the second cartridge containing a second pharmaceutical component; and
wherein the first and second cartridges are separated by a non-invasive valve component;
for the mixing and delivery of a first and second pharmaceutical component.

According to this aspect of the invention the non-invasive valve component is provided with a terminal rib to improve the seal formed between the non-invasive valve component and the second cartridge.

The key advantages of the dual cartridge pharmaceutical component-mixing delivery assembly of the present invention can be summarised as follows:
- Minimal air present in the reconstitution process
- Use of many standard components
- Diluent, Powder cartridges separate (processing in parallel, separate lines)
- Barrier properties
- Visible reconstitution
- Low waste/deadspace
- Minimal number of components in contact with the powder and diluent
- Less Air - removes priming step and enables auto-injection
- Minimal Stability/Drug interaction issues -
- Lower cost
- Plastic/glass combinations can be used
- Customisable for different pharmaceuticals
- Bulk lyophilisation of the solid component will be possible
- Reduced formulation burden
- Use of many conventional components
- Bulk testing before filling would be possible
- Multiple powders can be filled

The invention will now be illustrated by way of example only and with reference to the accompanying figures in which:
Figures 1 (a) to (d) are cross-sectional views of a pharmaceutical component-mixing delivery assembly comprising a ball valve component;
Figures 2 (a) to (d) are cross-sectional views of a pharmaceutical component-mixing delivery assembly comprising a ball valve component provided with ball displacement means;
Figures 3 (a) to (e) are cross-sectional views of a pharmaceutical component-mixing delivery assembly comprising a T-valve component;
Figures 4 (a) to (e) are cross-sectional views of a pharmaceutical component-mixing delivery assembly comprising an "annular valve" component;
Figures 5 (a) to (d) are cross-sectional views of a sealed pharmaceutical component-mixing delivery assembly comprising a partially threaded plunger and "needle valve" component;
Figures 6 (a) to (d) are cross-sectional views of a pharmaceutical component-mixing delivery assembly comprising a spigot plug component;
Figures 7 (a) and 7 (b) are cutaway perspective views of a pharmaceutical component-mixing delivery assembly comprising a spigot plug component of Figure 6; and Figure 7 (c) is a perspective view of the delivery assembly of Figure 6;
Figures 8 (a) to (d) are cross-sectional views of a pharmaceutical component-mixing delivery assembly comprising a hollow plug component;
Figures 9 (a) and 9 (b) are cutaway perspective views of a pharmaceutical component-mixing delivery assembly comprising a hollow plug component of Figure 8; Figure 9 (c) is a perspective view of the delivery assembly of Figure 8;
Figures 10 (a) and (b) are cross-sectional views of a pharmaceutical component-mixing delivery assembly comprising a ribbed non-invasive valve component;
Figures 11 (a) and 11 (b) are cutaway perspective views of a pharmaceutical component-mixing delivery assembly comprising ribbed non-invasive valve component of Figure 10; and
Figure 11 (c) is a perspective view of the delivery assembly of Figure 10.

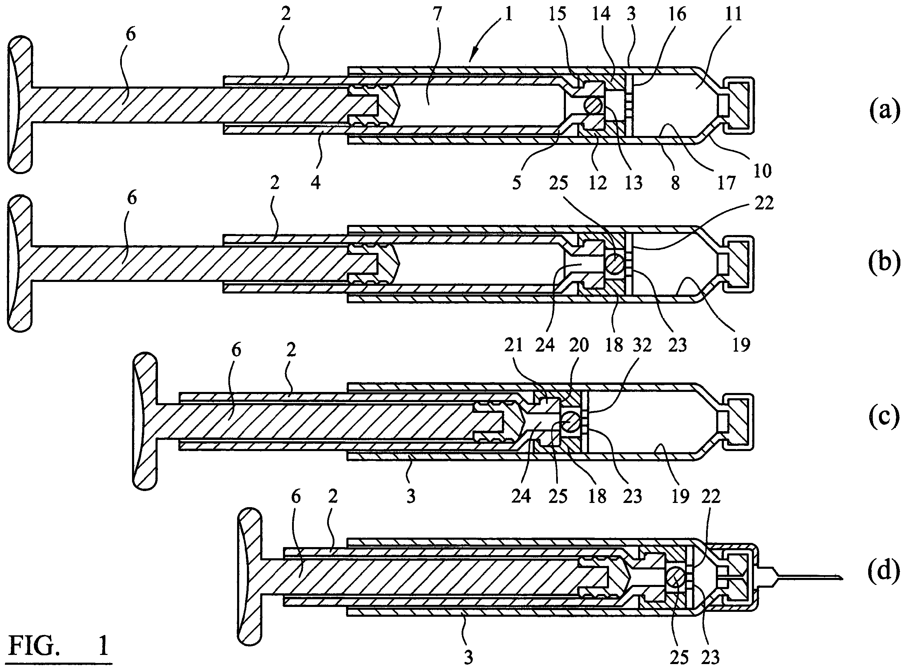

Referring to Figures 1 (a) to (d) a pharmaceutical component-mixing delivery assembly (1) comprises a first cartridge (2) and a second cartridge (3), the first cartridge (2) being dimensioned so that it is slidable within the second cartridge (3) from a pre-mixed position to a post-mixed position. The first cartridge (2) comprises a body (4) provided with an outlet end (5) and a plunger (6). The first cartridge (2) contains a first pharmaceutical component (7). The second cartridge (3) comprises a body (8) provided with an inlet end (17) and an outlet end (10). The second cartridge (3) contains a second pharmaceutical component (11). A non-invasive valve component (12) separates the first and second cartridges (2) and (3).

The non-invasive valve component (12) comprises a ball valve (13) which consists of a hollow piston (14) with a first end (15) adjacent the outlet end (5) of the first cartridge (2) and a second end (16) adjacent the inlet end (17) of the second cartridge (3). The hollow piston (14) comprises a side wall (18) which generally forms a seal against the inner surface (19) of the body (8) of the second cartridge (3).

The first end (15) of the hollow piston (14) is provided with a circumferential recess (20) suitable for locating the outlet end (5) of the first cartridge (2). In the embodiment shown the outlet end (5) of the first cartridge (2) is provided with a circumferential shoulder (21) adapted to fit into the circumferential recess (20) of the first end (15) of the hollow piston (14). The second end (16) of the hollow piston (14) is provided with a pair of apertures (22) and (23). The outlet end (5) of the first cartridge (2) is provided with an orifice (24) within which sits a ball (25) which acts to seal the first cartridge (2).

In use, when the plunger (6) is depressed within the first cartridge (2) the increase in pressure will force the ball (25) out of the orifice (24), which causes the valve component (12) to open allowing the first pharmaceutical component (7), usually a fluid to flow from the first cartridge (2) through orifice (24), around ball (25), through apertures (22) and (23) and into the second cartridge (3) where it mixes with the second pharmaceutical component (11).

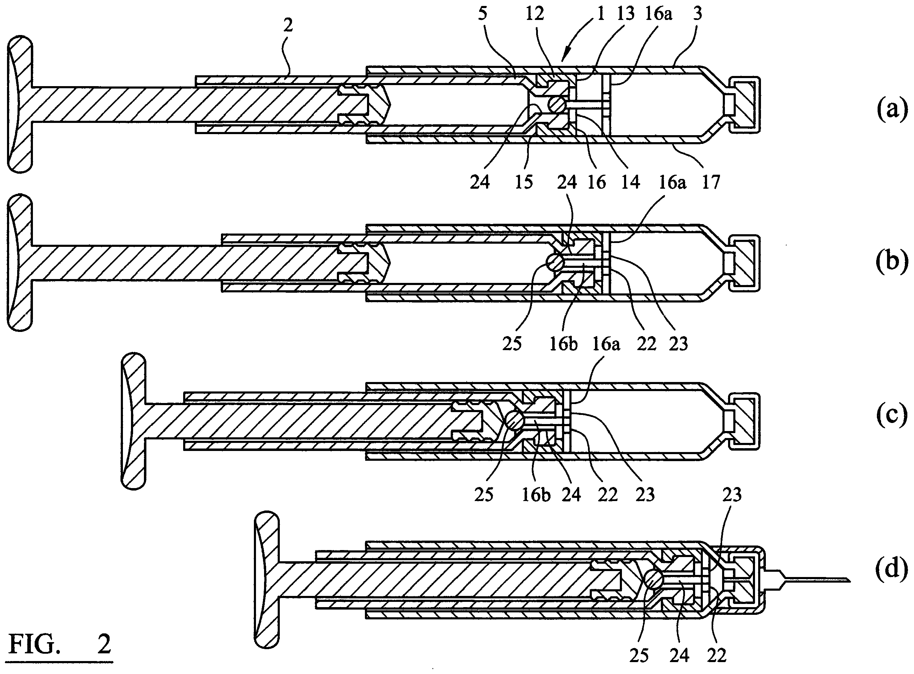

Referring to Figures 2 (a) to (d) a pharmaceutical component-mixing delivery assembly (1) comprises a first cartridge (2) and a second cartridge (3) as hereinbefore described with reference to Figures 1 (a) to (d). The non-invasive valve component (12) comprises a ball valve (13) which consists of a hollow piston (14) with a first end (15) adjacent the outlet end (5) of the first cartridge (2) and a second end (16) adjacent the inlet end (17) of the second cartridge (3) as hereinbefore described.

However, the second end (16) of the hollow piston (14) comprises a separate plug component (16a) which is provided with a pair of apertures (22) and (23). In addition, the plug component (16a) is provided with a substantially central spigot (16b) which protrudes from plug component (16a) towards the outlet end (5) of the first cartridge (2).

In use, when the plunger (6) is depressed within the first cartridge (2) the first cartridge (2) slides towards the plug component (16a). When the spigot (16b) comes into contact with the ball (25), the ball (25) is displaced from orifice (24), which causes the valve component (12) to open allowing the first pharmaceutical component (7), usually a fluid to flow from the first cartridge (2) through orifice (24), around ball (25), through apertures (22) and (23) and into the second cartridge (3) where it mixes with the second pharmaceutical component (11).

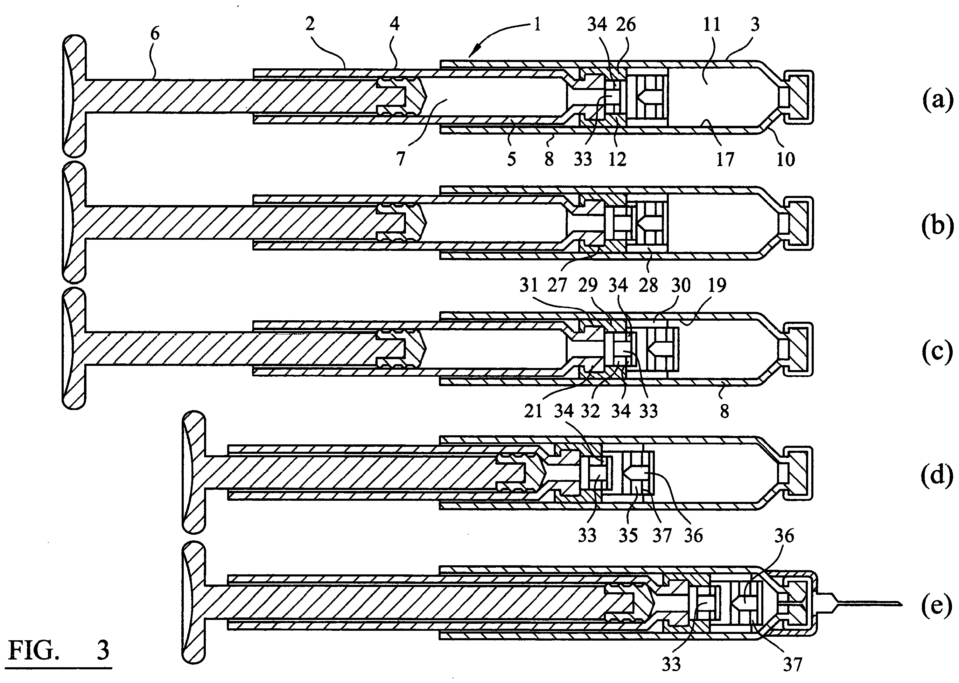

Referring to Figures 3 (a) to (e), a pharmaceutical component-mixing delivery assembly (1) comprises a first cartridge (2) and a second cartridge (3), the first cartridge (2) being dimensioned so that it is slidable within the second cartridge (3) from a pre-mixed position to a post-mixed position. The first cartridge (2) comprises a body (4) provided with an outlet end (5) and a plunger (6). The first cartridge (2) contains a first pharmaceutical component (7). The second cartridge (3) comprises a body (8) provided with an inlet end (17) and an outlet end (10). The second cartridge (3) contains a second pharmaceutical component (11). A non-invasive valve component (12) separates the first and second cartridges (2) and (3).

The non-invasive valve component (12) comprises a T-valve component (26). The T-valve component (26) consists of a pair of hollow pistons (27) and (28). A first hollow piston (27) is adjacent the outlet end (5) of the first cartridge (2) and a second hollow piston (28) is adjacent the inlet end (17) of the second cartridge (3). Each of the hollow pistons (27) and (28) comprises a side wall (29) and (30) respectively which generally forms a seal against the inner surface (19) of the body (8) of the second cartridge (3).

The first hollow piston (27) is provided with a circumferential recess (31) suitable for locating the outlet end (5) of the first cartridge (2). In the embodiment shown the outlet end (5) of the first cartridge (2) is provided with a circumferential shoulder (21) adapted to fit into the circumferential recess (31) of the first hollow piston (27). The first hollow piston (27) is fitted with a stopper component (32) with an internal conduit (33) which is substantially coaxial with the longitudinal axis of the first cartridge (2). The internal conduit (33) is provided with a branch (34) which seals against the side wall (29) of the hollow piston (27).

The second hollow piston (28) is fitted with a second stopper component (35) with an internal conduit (36) which is substantially coaxial with the longitudinal axis of the first cartridge (2). The internal conduit (36) is provided with a branch (37) which seals against the side wall (30) of the hollow piston (28).

In use, when the plunger (6) is depressed within the first cartridge (2) the increase in pressure will force the stopper component (32) to protrude out of the first hollow piston (27). This movement causes the branched conduit (34) to be exposed and lose its seals against the side wall (29) of the hollow piston (27) allowing the first pharmaceutical component (7), usually a fluid, to flow from the first cartridge (2) through internal conduit (33) and branched conduit (34) into the second hollow piston (28). Continued depression of the plunger (6) causes the second stopper component (35) to protrude out of the second hollow piston (28). This movement causes the branched conduit (37) to be exposed and lose its seal against the side wall (30) of the second hollow piston (28) allowing the first pharmaceutical component (7) to flow from the first cartridge (2) and first hollow piston (27) through internal conduit (36) and branched conduit (37) into the second cartridge (3) where it mixes with the second pharmaceutical component (11).

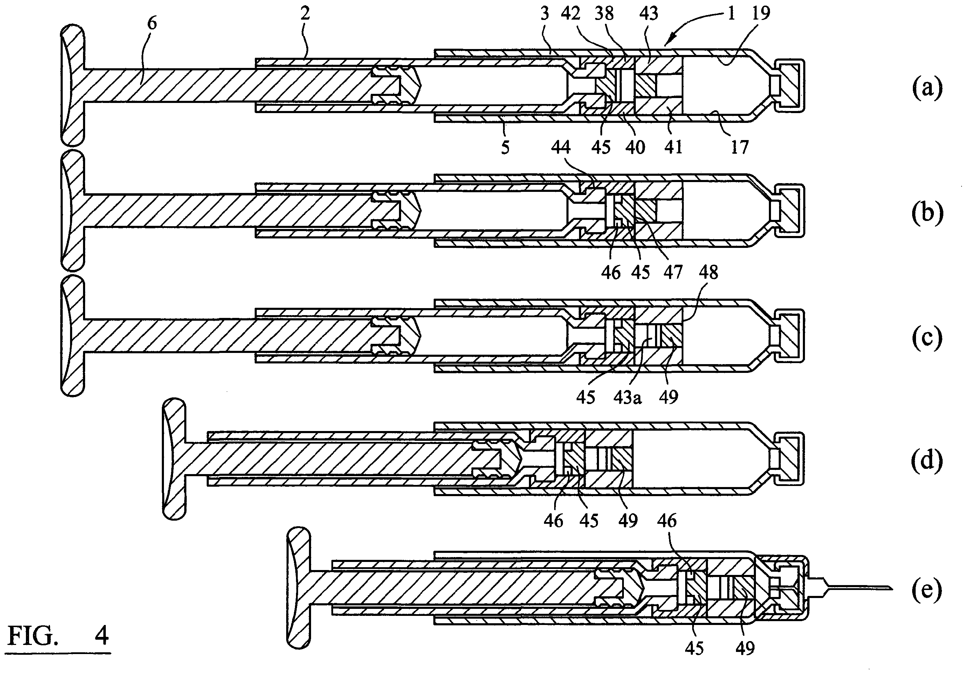

Referring to Figures 4 (a) to (e), a pharmaceutical component-mixing delivery assembly (1) comprises first and second cartridges (2) and (3) as hereinbefore described.

An annular valve component (38) comprises a round stopper component (39) with a conduit external to the stopper. This is hereinafter referred to an "annular valve".

The "annular valve" component (38) consists of a pair of hollow pistons (40) and (41). A first hollow piston (40) is adjacent the outlet end (5) of the first cartridge (2) and a second hollow piston (41) is adjacent the inlet end (17) of the second cartridge (3). Each of the hollow pistons (40) and (41) comprises a side wall (42) and (43) respectively which generally forms a seal against the inner surface (19) of the body (8) of the second cartridge (3).

The first hollow piston (40) is provided with a circumferential recess (44) suitable for locating the outlet end (5) of the first cartridge (2) The first hollow piston (40) is fitted with a first stopper component (45) with a circumferential shoulder (46) adapted to fit into the outlet end (5) of the first cartridge (2) and seal it.

The first hollow piston (40) comprises a star shaped orifice (47) whilst the first stopper component (45) is substantially circular in cross section.

Similarly, the second hollow piston (41) comprises a star shaped orifice (48) and is fitted with a second stopper component (49). The second stopper component (49) is substantially circular in cross section. However, the inner surface (43a) of the side wall (43) is provided with ridges (not shown) which enable the second stopper component (49) to form a seal.

It will be understood by the person skilled in the art that the shape of the orifices (47) and (48) and/or the first and second stopper components (45) and (49) may suitably be varied provided that the stopper (45) and (49) does not form a sealing fit within the orifice (47) and (48).

In use, when the plunger (6) is depressed within the first cartridge (2) the increase in pressure will force the first stopper component (45) out of the first cartridge (2). This movement allows the first pharmaceutical component (7), usually a fluid, to flow from the first cartridge (2) between the stopper component (45) and the side wall (42) of the piston (40) and through first orifice (47) into the second hollow piston (41). Continued depression of the plunger (6) causes the second stopper component (49) to move axially within the second hollow piston (41) away from the ridges (not shown). This movement causes the second stopper component (49) to lose its seal against the side wall (43) of the second hollow piston (41) allowing the first pharmaceutical component (7) to flow from the first cartridge (2) and first hollow piston (40) past the second stopper component (49) and through orifice (48) into the second cartridge (3) where it mixes with the second pharmaceutical component (11).

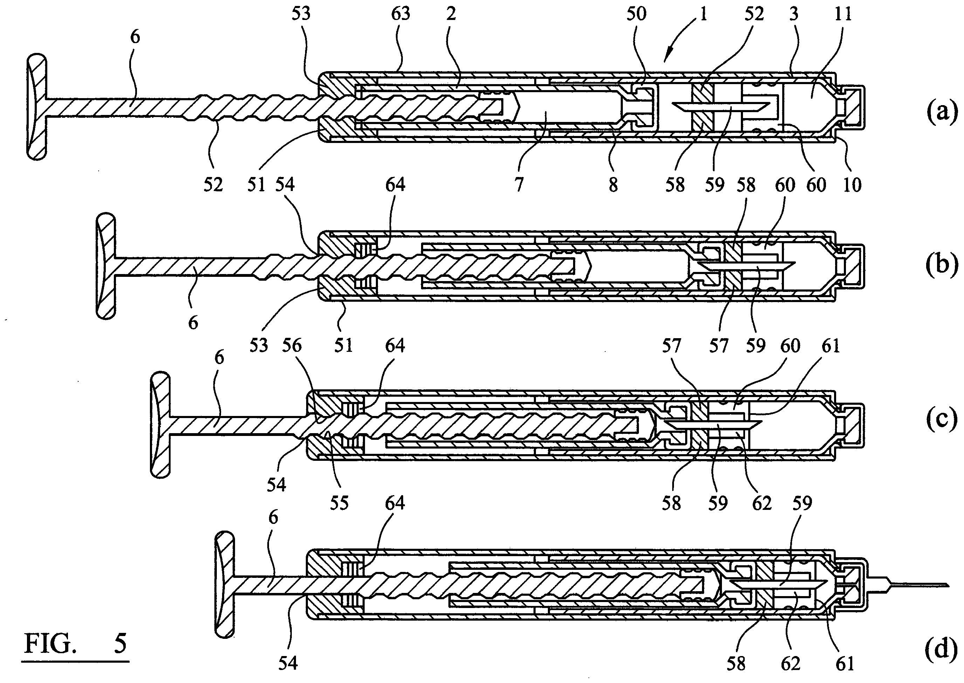

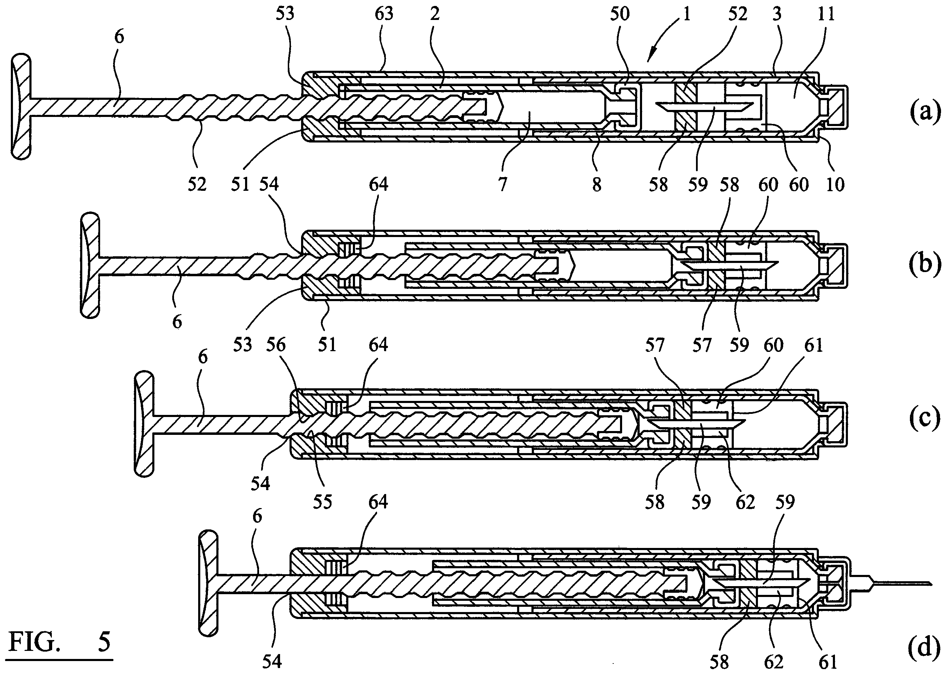

Referring to Figures 5 (a) to (d) a pharmaceutical component-mixing delivery assembly (1) comprises a first cartridge (2) and a second cartridge (3), the first cartridge (2) being dimensioned so that it is slidable within the second cartridge (3) from a pre-mixed position to a post-mixed position. An outer casing (63) is provided which houses the first and second cartridges (2 and 3). The first cartridge (2) comprises a body (4) provided with a sealed outlet end (50). The outer casing (63) comprises a sealed plunger end (51) and the plunger (6) protrudes through the sealed plunger end (51) of the outer casing (63). The first cartridge (2) contains a first pharmaceutical component (7). The second cartridge (3) comprises a body (8) provided with an inlet end (9) and an outlet end (10). The second cartridge (3) contains a second pharmaceutical component (11).

The plunger (6) is provided with a threaded surface (52) such that the threaded surface (52) is present along a predetermined portion of the length of the plunger (6). The sealed plunger end (51) of the outer casing (63) is provided with a stopper (53) which includes an orifice (54) through which the plunger (6) passes and a frangible seal (64). The inner surface (55) of the orifice (54) is provided with a threaded surface (56) which corresponds to and engages with the threaded surface (52) of the plunger (6). A plug component (57) separates the first and second cartridges (2) and (3). Said plug component (57) comprises a first seal (58) provided with a hollow needle (59) which extends either side of the first seal (58). A second seal (60) comprises a septum (61) provided with a guide channel (62) which, in use, is adapted to receive the needle (59) extending from the first seal (58).

In use, when the plunger (6) is turned within the orifice (54) of the stopper (53) in the outer casing (63) the frangible seal (64) is broken and the plunger (6) and the first cartridge (2) are pushed towards the second cartridge (3), causing the first cartridge (2) to disengage from the frangible seal (64). Continued turning of the plunger causes the first cartridge (2) to slide towards the plug component (57) until the needle (59) pierces the sealed outlet end (50) of the first cartridge (2). Continued turning of the plunger (6) causes the needle (59) to pierce the septum (61) allowing the first pharmaceutical component (7), usually a fluid, to flow from the first cartridge (2) through the needle (59) and into the second cartridge (3) where it mixes with the second pharmaceutical component (11). Once the first and second pharmaceutical components (7) and (11) are mixed and the threaded portion (52) of the plunger (6) has passed through the orifice (54), the plunger (6) may be pushed in a conventional manner to administer the medicament solution to a patient.

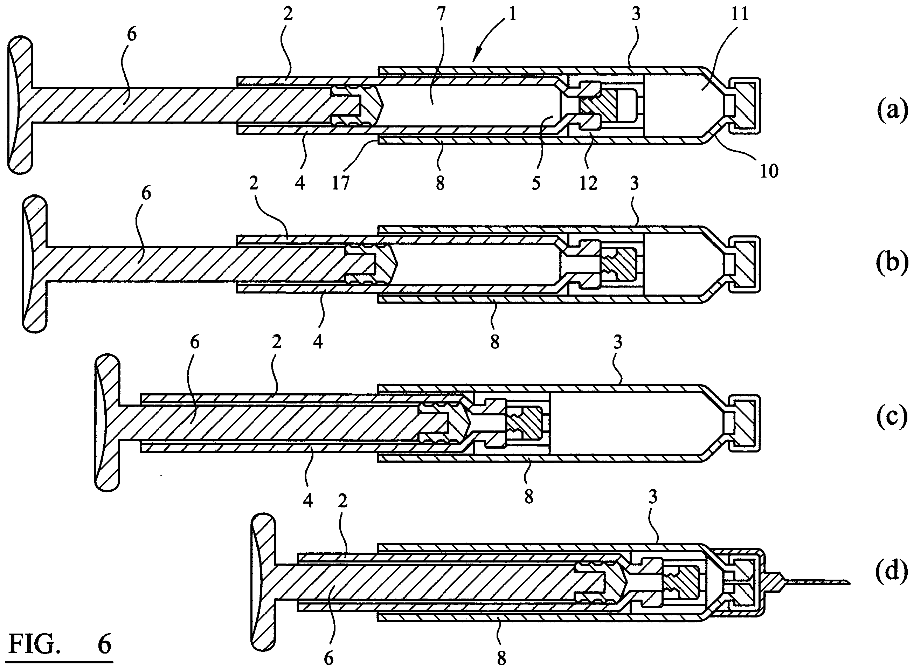

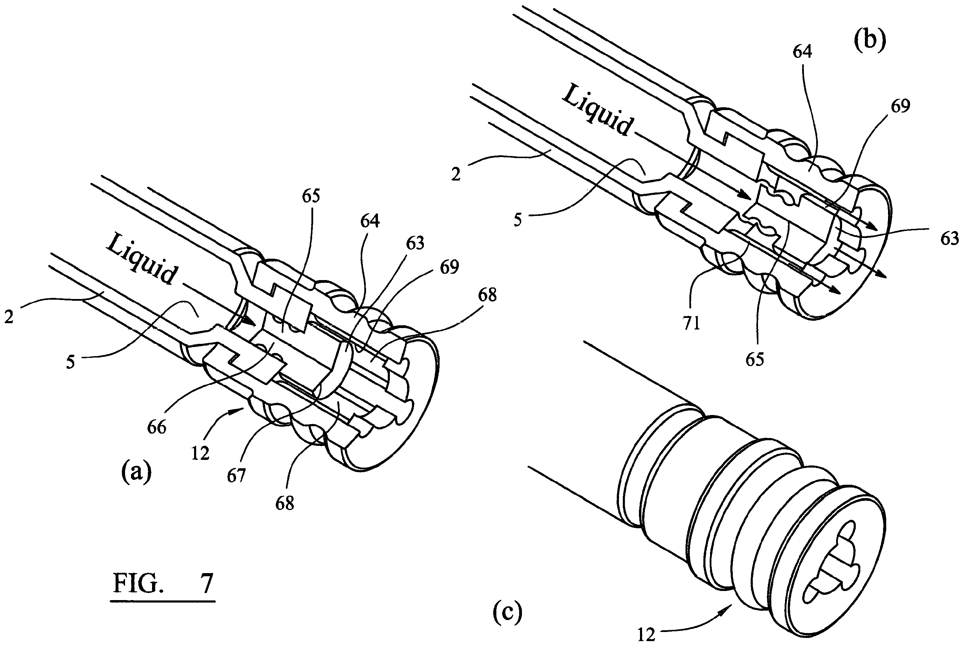

Referring to Figures 6 and 7 a pharmaceutical component-mixing delivery assembly (1) comprises a first cartridge (2) and a second cartridge (3), the first cartridge (2) being dimensioned so that it is slidable within the second cartridge (3) from a pre-mixed position to a post-mixed position. The first cartridge (2) comprises a body (4) provided with an outlet end (5) and a plunger (6). The first cartridge (2) contains a first pharmaceutical component (7). The second cartridge (3) comprises a body (8) provided with an inlet end (17) and an outlet end (10). The second cartridge (3) contains a second pharmaceutical component (11). A non-invasive valve component (12) separates the first and second cartridges (2) and (3).

The non-invasive valve component (12) comprises a spigot plug component (63) housed in a piston component (64). The piston component (64) is anchored to the first cartridge (2) by a lip (70). The spigot plug component (63) consists of a T-shaped component (65) with a first end (66) of narrower diameter forming a plug in the outlet end (5) of the first cartridge (2) and a second end (67) of wider diameter adjacent the inlet end (17) of the second cartridge (3). The dimensions of the second end (67) are such that it forms an "annular valve" component (68) with the side wall (69) of piston component (64).

In use, when the plunger (6) is depressed within the first cartridge (2) the increase in pressure will force the spigot plug component (63) away from outlet end (5) of the first cartridge (2), which creates a conduit (71) between T-shaped component (65) and the side wall (69) of piston component (64) through which the first pharmaceutical component (7), usually a fluid, can flow into the second cartridge (3) where it mixes with the second pharmaceutical component (11).

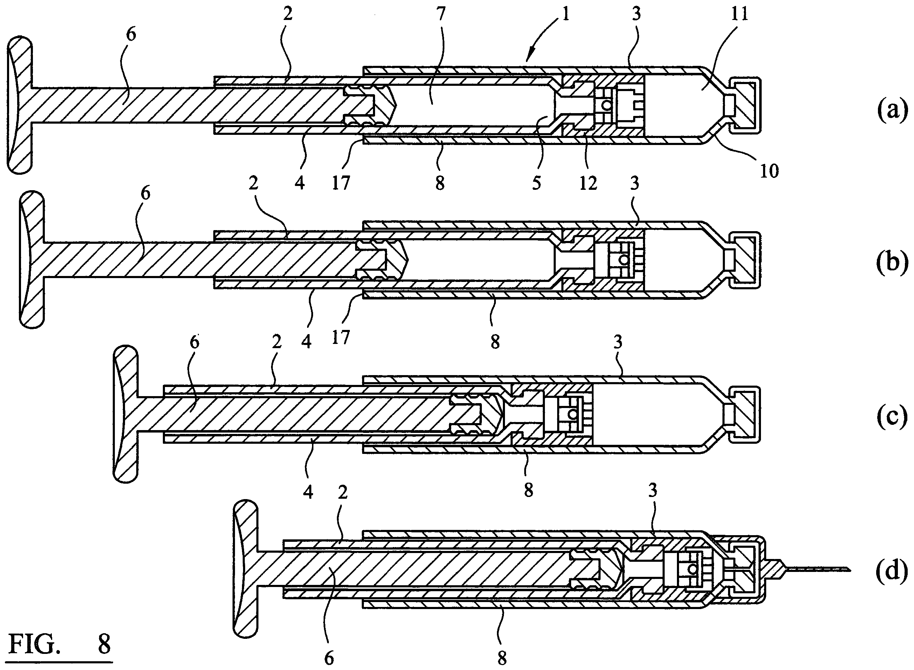

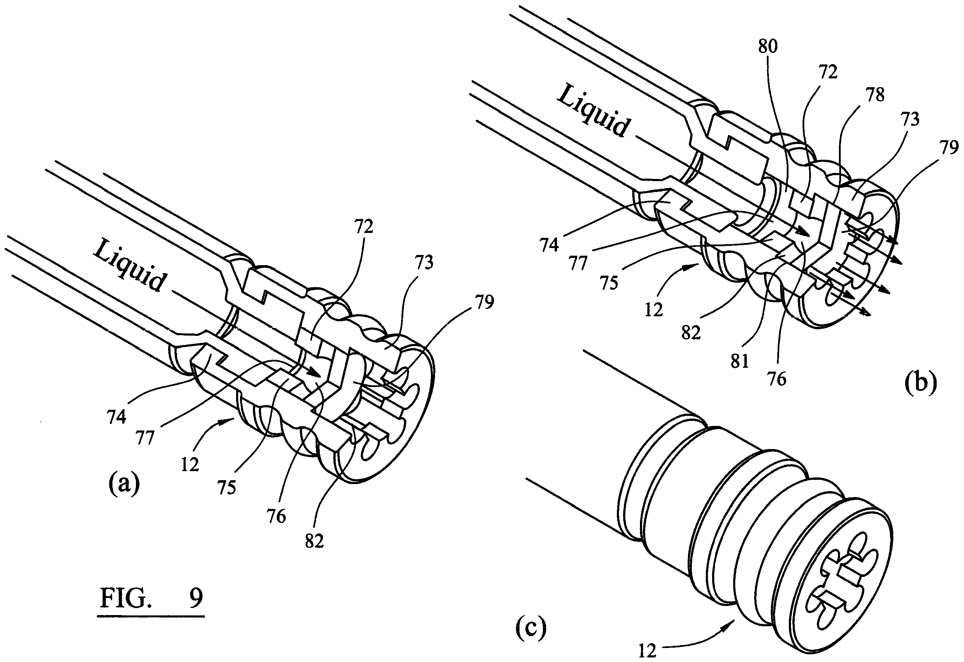

Referring to Figures 8 and 9 a pharmaceutical component-mixing delivery assembly (1) comprises a first cartridge (2) and a second cartridge (3), the first cartridge (2) being dimensioned so that it is slidable within the second cartridge (3) from a pre-mixed position to a post-mixed position. The first cartridge (2) comprises a body (4) provided with an outlet end (5) and a plunger (6). The first cartridge (2) contains a first pharmaceutical component (7). The second cartridge (3) comprises a body (8) provided with an inlet end (17) and an outlet end (10). The second cartridge (3) contains a second pharmaceutical component (11). A non-invasive valve component (12) separates the first and second cartridges (2) and (3).

The non-invasive valve component (12) comprises a hollow plug component (72) housed in a piston component (73). The piston component (73) is anchored to the first cartridge (2) by a lip (74). The hollow plug component (72) consists of a stopper (75) provided with a T-shaped conduit (76) such that the inlet end (77) of the conduit is coaxial with the first and second cartridges (2 and 3) and the outlet end (78) of the conduit is perpendicular to the inlet end (77) of the conduit, i.e. diametrical to the stopper (75). The piston component (73) is also T-shaped such that the outlet end (79) of the piston component is of greater diameter than the inlet end (80) of the piston component (73). Thus, the outlet end (78) of the conduit is capable of forming an "annular valve" component (81) with the side wall (82) of piston component (73).

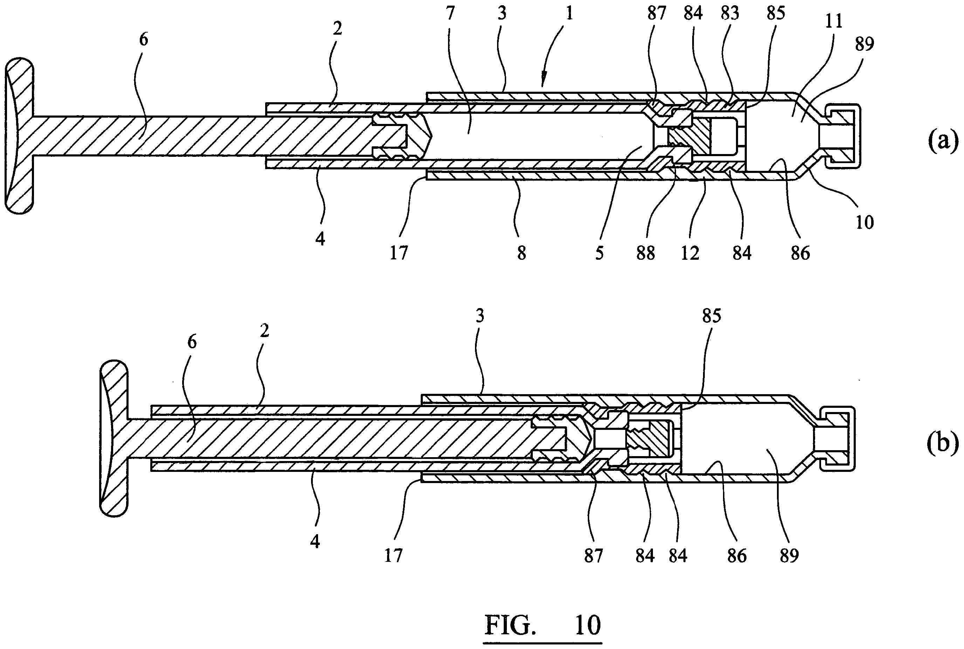

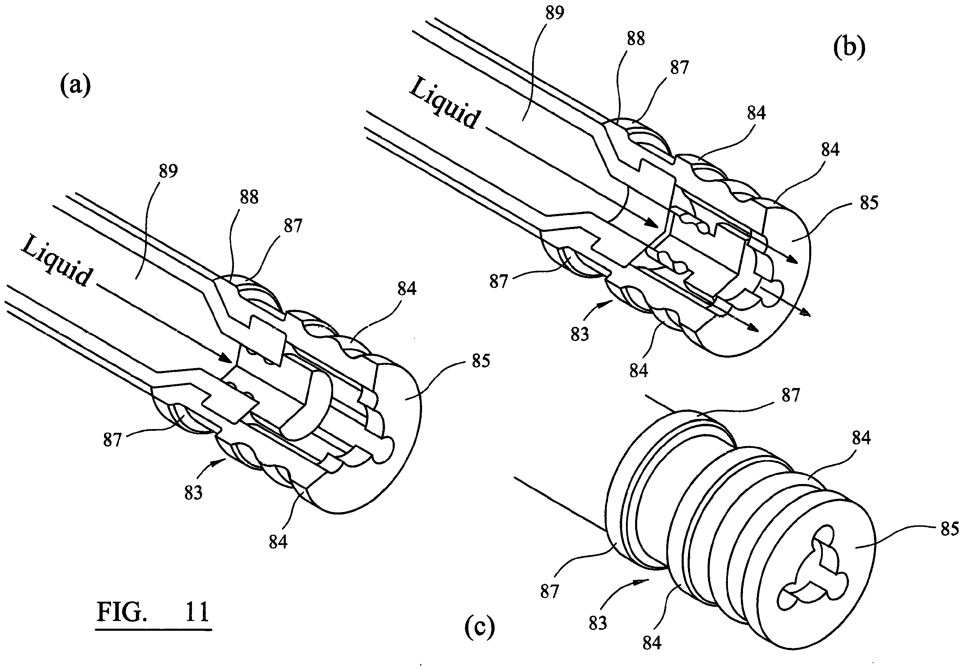

In use, when the plunger (6) is depressed within the first cartridge (2) the increase in pressure will force the hollow plug component (72) away from outlet end (5) of the first cartridge (2), which opens the T-shaped conduit (76). Thus, the first pharmaceutical component (7), usually a fluid, can flow through the inlet end (77) of the conduit to the outlet end (78) of the conduit and between the side of the hollow plug component (72) and the side wall (82) of piston component (73) into the second cartridge (3) where it mixes with the second pharmaceutical component (11). Referring to Figures 10 and 11 a pharmaceutical component-mixing delivery assembly (1) comprises a first cartridge (2) and a second cartridge (3), the first cartridge (2) being dimensioned so that it is slidable within the second cartridge (3) from a pre-mixed position to a post-mixed position. The first cartridge (2) comprises a body (4) provided with an outlet end (5) and a plunger (6). The first cartridge (2) contains a first pharmaceutical component (7). The second cartridge (3) comprises a body (8) provided with an inlet end (17) and an outlet end (10). The second cartridge (3) contains a second pharmaceutical component (11). A non-invasive valve component (12) separates the first and second cartridges (2) and (3).

The non-invasive valve component (12) comprises an elongated piston (83). The piston (83) is provided with a plurality of external circumferential ribs (84) which form a seal between the side wall (85) of the piston (83) and the inner wall (86) of the second cartridge (3). The piston is also provided with terminal rib (87), comprising a circumferential rib located at the end (88) of the piston (83) nearest to the inlet end (17) of the second cartridge (3).

The region of the second cartridge (3) that contains the second pharmaceutical component (11) will generally comprise an aseptic mixing chamber (89). The length of the piston (83) will be dictated by how far back along the second cartridge (3) it has to move, which will in turn be dictated by the volume required for the mixing chamber (89). In use, as the plunger (6) is depressed and the piston (83) moves towards the inlet end (17) of the second cartridge (3), the mixing chamber (89) does not come into contact with the non-aseptic inner wall (86) of the inlet end (17) of the second cartridge (3) and the sterility of the mixing chamber (89) is maintained at all times.

## Claims

1. A pharmaceutical component-mixing delivery assembly (1) comprising:
a first cartridge (2) and a second cartridge (3);
the first and second cartridges (2 and 3) being dimensioned so that they are movable relative to one another such that the first cartridge (2) is slidable within the second cartridge (3) from a pre-mixed position to a post-mixed position;
the first cartridge (2) comprising a body (4) provided with an outlet end (5), the first cartridge (2) being fitted with a plunger (6) and containing a first pharmaceutical component (7);
the second cartridge (3) comprising a body (8) provided with an inlet end (17) and an outlet end (10), the second cartridge (3) comprising an aseptic mixing chamber (89) and containing a second pharmaceutical component (11); wherein the first cartridge (2) and the second cartridge (3) are separated by a valve component (12) comprising a piston (83) so that as the plunger (6) is depressed, the piston (83) moves towards the inlet (17) of the second cartridge (3) and the mixing chamber (89) expands longitudinally and
**characterised in that** the valve component (12) is a non-invasive valve component and the piston (83) is provided with a terminal rib (87), comprising a circumferential rib located at the end (88) of the piston (83) nearest to the inlet end (17) of the second cartridge (3) for providing a seal between the side wall (85) of the piston and the inner wall (86) of the second cartridge (3); and wherein the inner wall (86) of the second cartridge (3), outside of the mixing chamber (89) and beyond the terminal rib (87) of the piston (83), is non-aseptic; the non-invasive valve component (12) is generally elongated, the length of which is chosen so as to ensure that, as the mixing chamber (89) expands longitudinally, the pharmaceutical components (7 and 11) never come into contact with the non-aseptic wall of the second cartridge (3).

2. A pharmaceutical component-mixing delivery assembly (1) according to claim 1 wherein the non-invasive valve component (12) comprises a T-shaped stopper component or a ball valve (13); and a flow through stopper component.

3. A pharmaceutical component-mixing delivery assembly (1) according to any one of the preceding claims wherein the assembly comprises a syringe assembly.

4. A pharmaceutical component-mixing delivery assembly (1) according to any one of the preceding claims wherein one of the first and second pharmaceutical components (7 and 11) comprises a solid or liquid therapeutically active agent and the other is a liquid capable of solubilising the solid therapeutically active agent or capable of mixing with the liquid therapeutically active agent.

5. A pharmaceutical component-mixing delivery assembly (1) according to claim 2 wherein the ball valve component (13) comprises one or more balls located within the outlet of the first cartridge (2), such that the ball(s) acts to seal the first cartridge (2).

6. A pharmaceutical component-mixing delivery assembly (1) according to claim 6 wherein the ball valve component (13) includes a plug component (16a) which is provided with one or more apertures (22 and 23) and which is provided with means for displacing the ball from the outlet of the first cartridge (2).

7. A pharmaceutical component-mixing delivery assembly (1) according to any one of claims 1 to 6 wherein the non-invasive valve component (12) comprises a flow through stopper component.

8. A pharmaceutical component-mixing delivery assembly (1) according to claim 7 wherein the flow through stopper component is an "annular valve" (38).

9. A pharmaceutical component-mixing delivery assembly (1) according to claim 8 wherein the flow through stopper component comprises a hollow annular valve component and a T-shaped plug component (65) such that the outlet end (5) of the plug component is of greater diameter than the inlet end (17) of the piston component (64).

10. A method of operating a pharmaceutical component-mixing delivery assembly (1) holding first and second pharmaceutical components (7 and 11); said delivery assembly comprising:
a first cartridge (2) and a second cartridge (3);
the first and second cartridges (2 and 3) being dimensioned so that they are movable relative to one another such that the first cartridge (2) is slidable within the second cartridge (3) from a pre-mixed position to a post-mixed position;
the first cartridge (2) comprising a body (4) provided with an outlet end (5), the first cartridge (2) being fitted with plunger (6) and containing a first pharmaceutical component (7);
the second cartridge (3) comprising a body (8) provided with an inlet end (17) and an outlet end (10), the second cartridge (3) comprising an aseptic mixing chamber (89) and containing a second pharmaceutical component (11); wherein the first cartridge (2) and the second cartridge (3) are separated by a valve component (12) comprising a piston (83) so that as the plunger (6) is depressed, the piston (83) moves towards the inlet (17) of the second cartridge (3) and the mixing chamber (89) expands longitudinally and **characterised in that** the valve component (12) is a non-invasive valve component and the piston (83) is provided with a terminal rib (87), comprising a circumferential rib located at the end (88) of the piston (83) nearest to the inlet end (17) of the second cartridge (3) for providing a seal between the side wall (18, 85) of the piston and the inner wall (19, 86) of the second cartridge (3), to improve the seal formed between the non-invasive valve component (12) and the second cartridge (3); the inner wall (86) of the second cartridge (3), outside of the mixing chamber and beyond the terminal rib (87) of the piston (83), is non-aseptic; and wherein the length of the valve component (12) is chosen so as to ensure that, as the mixing chamber (89) expands longitudinally, the pharmaceutical components (7 and 11) never come into contact with the non-aseptic wall of the second cartridge (3);
said method comprising pressing the plunger (6) axially into the first cartridge (2) to an intermediate position thus pressurizing the first cartridge (2) and opening the non-invasive valve component (12), causing the first pharmaceutical component (7) to flow through the valve and into the second cartridge (3) to mix with the second pharmaceutical component (11).

## Patentansprüche

1. Abgabeanordnung (1) zum Mischen von pharmazeutischen Komponenten, umfassend:
eine erste Kartusche (2) und eine zweite Kartusche (3);
wobei die erste und zweite Kartusche (2 und 3) derart bemessen sind, dass sie bezogen aufeinander bewegt werden können, sodass die erste Kartusche (2) innerhalb der zweiten Kartusche (3) aus einer Position vor dem Mischen in eine Position nach dem Mischen verschoben werden kann;
wobei die erste Kartusche (2) einen Körper (4) umfasst, der mit einem Auslassende (5) bereitgestellt ist, wobei die erste Kartusche (2) mit einem Schieber (6) ausgestattet ist und eine erste pharmazeutische Komponente (7) enthält;
wobei die zweite Kartusche (3) einen Körper (8) umfasst, der mit einem Einlassende (17) und einem Auslassende (10) bereitgestellt ist, wobei die zweite Kartusche (3) eine aseptische Mischkammer (89) umfasst und eine zweite pharmazeutische Komponente (11) enthält; und
**dadurch gekennzeichnet, dass** die Ventilkomponente (12) eine nichtinvasile Ventilkomponente ist und der Kolben (83) mit einer Abschlussrippe (87) bereitgestellt ist, die eine Umfangsrippe umfasst, die sich an dem Ende (88) des Kolbens (83) am nächsten zu dem Einlassende (17) der zweiten Kartusche (3) befindet, um eine Abdichtung zwischen der Seitenwand (85) des Kolbens und der Innenwand (86) der zweiten Kartusche (3) bereitzustellen; und wobei die Innenwand (86) der zweiten Kartusche (3) außerhalb der Mischkammer (89) und jenseits der Abschlussrippe (87) des Kolbens (83) nicht aseptisch ist; wobei die nichtinvasive Ventilkomponente (12) im Allgemeinen länglich ist, wobei deren Länge derart ausgewählt ist, dass sichergestellt wird, dass die pharmazeutischen Komponenten (7 und 11) zu keiner Zeit mit der nichtaseptischen Wand der zweiten Kartusche (3) in Berührung kommen, während sich die Mischkammer (89) in Längsrichtung ausdehnt.

2. Abgabeanordnung (1) zum Mischen von pharmazeutischen Komponenten nach Anspruch 1, wobei die nichtinvasive Ventilkomponente (12) Folgendes umfasst: eine T-förmige Anschlagkomponente oder ein Kugelventil (13); und einen Ablauf durch die Anschlagkomponente.

3. Abgabeanordnung (1) zum Mischen von pharmazeutischen Komponenten nach einem der vorangehenden Ansprüche, wobei die Anordnung eine Spritzenanordnung umfasst.

4. Abgabeanordnung (1) zum Mischen von pharmazeutischen Komponenten nach einem der vorangehenden Ansprüche, wobei eine von der ersten und zweiten pharmazeutischen Komponente (7 und 11) einen festen oder flüssigen therapeutischen Wirkstoff umfasst und es sich bei der anderen um eine Flüssigkeit handelt, die in der Lage ist, den festen therapeutischen Wirkstoff zu solubilisieren, oder in der Lage ist, sich mit dem flüssigen therapeutischen Wirkstoff zu vermischen.

5. Abgabeanordnung (1) zum Mischen von pharmazeutischen Komponenten nach Anspruch 2, wobei die Kugelventilkomponente (13) eine oder mehrere Kugeln umfasst, die sich innerhalb des Auslasses der ersten Kartusche (2) befinden, sodass die Kugel(n) wirkt (wirken), um die erste Kartusche (2) abzudichten.

6. Abgabeanordnung (1) zum Mischen von pharmazeutischen Komponenten nach Anspruch 6, wobei die Kugelventilkomponente (13) eine Stopfenkomponente (16a) beinhaltet, die mit einer oder mehreren Öffnungen (22 und 23) bereitgestellt ist und die mit einem Mittel zum Verschieben der Kugel aus dem Auslass der ersten Kartusche (2) bereitgestellt ist.

7. Abgabeanordnung (1) zum Mischen von pharmazeutischen Komponenten nach einem der Ansprüche 1 bis 6, wobei die nichtinvasive Ventilkomponente (12) einen Ablauf durch die Anschlagkomponente umfasst.

8. Abgabeanordnung (1) zum Mischen von pharmazeutischen Komponenten nach Anspruch 7, wobei der Ablauf durch die Anschlagkomponente ein "ringförmiges Ventil" (38) ist.

9. Abgabeanordnung (1) zum Mischen von pharmazeutischen Komponenten nach Anspruch 8, wobei der Ablauf durch die Anschlagkomponente eine hohle ringförmige Ventilkomponente und eine T-förmige Stopfenkomponente (65) umfasst, sodass das Auslassende (5) der Stopfenkomponente einen größeren Durchmesser aufweist als das Einlassende (17) der Kolbenkomponente (64).

10. Verfahren zum Betätigen einer Abgabeanordnung (1) zum Mischen von pharmazeutischen Komponenten, die eine erste und zweite pharmazeutische Komponente (7 und 11) hält; wobei die Abgabeanordnung Folgendes umfasst:
eine erste Kartusche (2) und eine zweite Kartusche (3);
wobei die erste und zweite Kartusche (2 und 3) derart bemessen sind, dass sie bezogen aufeinander bewegt werden können, sodass die erste Kartusche (2) innerhalb der zweiten Kartusche (3) aus einer Position vor dem Mischen in eine Position nach dem Mischen verschoben werden kann;
wobei die erste Kartusche (2) einen Körper (4) umfasst, der mit einem Auslassende (5) bereitgestellt ist, wobei die erste Kartusche (2) mit einem Schieber (6) ausgestattet ist und eine erste pharmazeutische Komponente (7) enthält;
wobei die zweite Kartusche (3) einen Körper (8) umfasst, der mit einem Einlassende (17) und einem Auslassende (10) bereitgestellt ist, wobei die zweite Kartusche (3) eine aseptische Mischkammer (89) umfasst und eine zweite pharmazeutische Komponente (11) enthält; wobei die erste Kartusche (2) und die zweite Kartusche (3) durch eine Ventilkomponente (12) getrennt sind, die einen Kolben (83) umfasst, sodass sich der Kolben (83) in Richtung des Einlasses (17) der zweiten Kartusche (3) bewegt und sich die Mischkammer (89) in Längsrichtung ausweitet, wenn der Schieber (6) zusammengedrückt wird; und **dadurch gekennzeichnet, dass** die Ventilkomponente (12) ein nichtinvasiles Ventil ist und der Kolben (83) mit einer Abschlussrippe (87) bereitgestellt ist, die eine Umfangsrippe umfasst, die sich an dem Ende (88) des Kolbens (83) am nächsten zu dem Einlassende (17) der zweiten Kartusche (3) befindet, um eine Abdichtung zwischen der Seitenwand (18, 85) des Kolbens und der Innenwand (19, 86) der zweiten Kartusche (3) bereitzustellen, um die Abdichtung zu verbessern, die zwischen der nichtinvasiven Ventilkomponente (12) und der zweiten Kartusche (3) gebildet ist; wobei die Innenwand (86) der zweiten Kartusche (3) außerhalb der Mischkammer und jenseits der Abschlussrippe (87) des Kolbens (83) nicht aseptisch ist; und wobei die Länge der Ventilkomponente (12) derart ausgewählt ist, dass sichergestellt wird, dass die pharmazeutischen Komponenten (7 und 11) zu keiner Zeit mit der nichtaseptischen Wand der zweiten Kartusche (3) in Berührung kommen, während sich die Mischkammer (89) in Längsrichtung ausdehnt;
wobei das Verfahren Folgendes umfasst: Drücken des Schiebers (6) axial in die erste Kartusche (2) in eine Zwischenposition, wodurch die erste Kartusche (2) unter Druck gesetzt wird, und Öffnen der nichtinvasiven Ventilkomponente (12), wodurch die erste pharmazeutische Komponente (7) dazu veranlasst wird, durch das Ventil und in die zweite Kartusche (3) zu strömen, um sich mit der zweiten pharmazeutischen Komponente (11) zu vermischen.

## Revendications

1. Ensemble de délivrance mélangeant des composants pharmaceutiques (1) comprenant :
une première cartouche (2) et une deuxième cartouche (3) ;
les première et deuxième cartouches (2 et 3) étant dimensionnées de manière à être capables de mouvement l'une par rapport à l'autre de sorte que la première cartouche (2) est capable de coulissement au sein de la deuxième cartouche (3) d'une position d'avant mélange à une positon d'après mélange ;
la première cartouche (2) comprenant un corps (4) pourvu d'une extrémité de sortie (5), la première cartouche (2) étant munie d'un plongeur (6) et contenant un premier composant pharmaceutique (7) ;
la deuxième cartouche (3) comprenant un corps (8) pourvu d'une extrémité d'entrée (17) et d'une extrémité de sortie (10), la deuxième cartouche (3) comprenant une chambre de mélange aseptique (89) et contenant un deuxième composant pharmaceutique (11) ; et
**caractérisé en ce que** le composant de soupape (12) est un composant de soupape non invasif et le piston (83) est pourvu d'une nervure terminale (87), comprenant une nervure circonférentielle située au niveau de l'extrémité (88) du piston (83) la plus proche de l'extrémité d'entrée (17) de la deuxième cartouche (3) destinée à fournir un joint étanche entre la paroi latérale (85) du piston et la paroi interne (86) de la deuxième cartouche (3) ; et dans lequel la paroi interne (86) de la deuxième cartouche (3), à l'extérieur de la chambre de mélange (89) et au-delà de la nervure terminale (87) du piston (83), est non aseptique ; le composant de soupape non invasif (12) est allongé de manière générale, la longueur duquel est choisie de manière à garantir que, quand la chambre de mélange (89) s'étend longitudinalement, les composants pharmaceutiques (7 et 11) ne viennent jamais en contact avec la paroi non aseptique de la deuxième cartouche (3).

2. Ensemble de délivrance mélangeant des composants pharmaceutiques (1) selon la revendication 1 dans lequel le composant de soupape non invasif (12) comprend un composant de bouchon en forme de T ou une soupape à bille (13) ; et un composant de bouchon à traversée d'écoulement.

3. Ensemble de délivrance mélangeant des composants pharmaceutiques (1) selon l'une quelconque des revendications précédentes dans lequel l'ensemble comprend un ensemble de seringue.

4. Ensemble de délivrance mélangeant des composants pharmaceutiques (1) selon l'une quelconque des revendications précédentes dans lequel l'un des premier et deuxièmes composants pharmaceutiques (7 et 11) comprend un agent thérapeutiquement actif solide ou liquide et l'autre est un liquide capable de solubiliser l'agent thérapeutiquement actif solide ou capable de se mélanger avec l'agent thérapeutiquement actif liquide.

5. Ensemble de délivrance mélangeant des composants pharmaceutiques (1) selon la revendication 2 dans lequel le composant de soupape à bille (13) comprend une ou plusieurs billes situées au sein de la sortie de la première cartouche (2), de sorte que la/les bille(s) agit/agissent pour rendre la première cartouche (2) étanche.

6. Ensemble de délivrance mélangeant des composants pharmaceutiques (1) selon la revendication 6 dans lequel le composant de soupape à bille (13) inclut un composant d'obturateur (16a) qui est pourvu d'une ou de plusieurs ouvertures (22 et 23) et qui est pourvu d'un moyen destiné à déplacer la bille de la sortie de la première cartouche (2).

7. Ensemble de délivrance mélangeant des composants pharmaceutiques (1) selon l'une quelconque des revendications 1 à 6 dans lequel le composant de soupape non invasif (12) comprend un composant de bouchon à traversée d'écoulement.

8. Ensemble de délivrance mélangeant des composants pharmaceutiques (1) selon la revendication 7 dans lequel le composant de bouchon à traversée d'écoulement est une « soupape annulaire » (38).

9. Ensemble de délivrance mélangeant des composants pharmaceutiques (1) selon la revendication 8 dans lequel le composant de bouchon à traversée d'écoulement comprend un composant de soupape annulaire creuse et un composant d'obturateur en forme de T (65) de sorte que l'extrémité de sortie (5) du composant de bouchon a un diamètre plus grand que l'extrémité d'entrée (17) du composant de piston (64).

10. Procédé de fonctionnement d'un ensemble de délivrance mélangeant des composants pharmaceutiques (1) retenant les premier et deuxième composants pharmaceutiques (7 et 11) ; ledit ensemble de délivrance comprenant :
une première cartouche (2) et une deuxième cartouche (3) ;
les première et deuxième cartouches (2 et 3) étant dimensionnées de manière à être capables de mouvement l'une par rapport à l'autre de sorte que la première cartouche (2) est capable de coulissement au sein de la deuxième cartouche (3) d'une position d'avant mélange à une position d'après mélange ;
la première cartouche (2) comprenant un corps (4) pourvu d'une extrémité de sortie (5), la première cartouche (2) étant munie d'un plongeur (6) et contenant un premier composant pharmaceutique (7) ;
la deuxième cartouche (3) comprenant un corps (8) pourvu d'une extrémité d'entrée (17) et d'une extrémité de sortie (10), la deuxième cartouche (3) comprenant une chambre de mélange aseptique (89) et contenant un deuxième composant pharmaceutique (11) ; dans lequel la première cartouche (2) et la deuxième cartouche (3) sont séparées par un composant de soupape (12) comprenant un piston (83) de sorte que quand le plongeur (6) est enfoncé, le piston (83) se déplace vers l'entrée (17) de la deuxième cartouche (3) et la chambre de mélange (89) s'étend longitudinalement et **caractérisé en ce que** le composant de soupape (12) est une soupape non invasive et le piston (83) est pourvu d'une nervure terminale (87), comprenant une nervure circonférentielle située au niveau de l'extrémité (88) du piston (83) la plus proche de l'extrémité d'entrée (17) de la deuxième cartouche (3) destinée à fournir un joint étanche entre la paroi latérale (18, 85) du piston et la paroi interne (19, 86) de la deuxième cartouche (3), pour améliorer le joint étanche formé entre le composant de soupape non invasif (12) et la deuxième cartouche (3) ; la paroi interne (86) de la deuxième cartouche (3), à l'extérieur de la chambre de mélange et au-delà de la nervure terminale (87) du piston (83), est non aseptique ; et dans lequel la longueur du composant de soupape (12) est choisie de manière à garantir que, quand la chambre de mélange (89) s'étend longitudinalement, les composants pharmaceutiques (7 et 11) ne viennent jamais en contact avec la paroi non aseptique de la deuxième cartouche (3) ;
ledit procédé comprenant la pression du plongeur (6) de manière axiale dans la première cartouche (2) jusqu'à une position intermédiaire pressurisant ainsi la première cartouche (2) et ouvrant le composant de soupape non invasif (12), amenant le premier composant pharmaceutique (7) à s'écouler à travers la soupape et jusque dans la deuxième cartouche (3) pour se mélanger avec le deuxième composant pharmaceutique (11).
